# EUROPEAN PATENT APPLICATION

(11) **EP 1 795 127 A2**
(43) Date of publication of application: **13.06.2007**
(21) Application number: 06025466.1
(22) Date of filing: 08.12.2006
(51) Int. Cl.: A61B 5/15

(54) **Blood collection needle**

(30) Priority: 09.12.2005 JP 2005356805
(71) Applicant: Fujifilm Corporation, Minato-ku Tokyo (JP)
(72) Inventor: Tashiro, Tomoko FUJIFILM Corporation, Asaka-shi Saitama (JP); Sakaino, Yoshiki FUJIFILM Corporation, Asaka-shi Saitama (JP); Abe, Yoshihiko FUJIFILM Corporation, Asaka-shi Saitama (JP)
(74) Representative: Banzer, Hans-Jörg

(57) **Abstract**

A blood collecting needle, which comprises: a needle hub (b); a human-body puncturing needle tube (a) provided at one side of the needle hub; and a plug-body puncturing needle tube (c) provided at the other side of the needle hub, wherein an outer diameter of a tip (d) of the human-body puncturing needle tube (a) and an outer diameter of a tip (e) of the plug-body puncturing needle tube (c) are different from each other.

## Description

### Background of the Invention

### 1. Field of the Invention

The present invention relates to a blood collecting needle, and more particularly to a test kit and a blood collecting kit each of which is equipped with the blood collecting needle.

### 2. Description of the Related Art

A method of diagnosing diseases of a human by using blood, urine or the like as a test substance has been hitherto executed as a method that can simply perform diagnosis without damaging human bodies.

Particularly, blood can be diagnosed with respect to many test items, and with respect to health examination, diagnosis of diseases, etc., it is general to collect blood and analyze the components of the blood.

In the blood examination, a blood collecting needle is stung into vein when blood is collected from a so-called a person being examined such as a patient, a health examination target or the like. In the normal blood examination, 21G (gauge) defined in ISO9626:1991 or the like (outer diameter of 0.81mm, inner diameter of 0.49mm to 0.61mm) is used, and it is normal that a human is accompanied by pain when a needle is stung into the vein of the human, and thus it has been required in the medical field that all efforts are exerted to eliminate pain when blood is collected.

As a means of solving the needle-sting problem described above, JP-A-2003-83958 has disclosed a technique of collecting blood with a needle having a small outer diameter so that pain is suppressed.

Furthermore, JP-A-57-11661hasproposed a blood collecting needle having high safety that can check sting of the needle into vein by joining two needle tubes through a needle base portion.

### Summary of the Invention

The very narrow needle disclosed in the JP-A-2003-83958 has a problem that it is difficult to sting the needle into a human body.

Furthermore, according to a double-ended needle used for general blood collection, one side of the needle is stung into a human body and the other side is stung through the plug body of a blood collecting tube. Therefore, when the needle is narrowed to suppress pain, the needle may be broken or bent when it is stung through the plug body.

In the technique of the JP-A-57-11661, two needle tubes are joined to each other in order to check the flow-in of blood. The patent concerned has no description concerning the dimension of the two needle tubes, and also has no mention about the problem of the sting through the plug body of the blood collecting tube occurring in the case of a needle having a small outer diameter.

Furthermore, even in the case of a injection needle which is normally used for syringe blood collection, vacuum blood collection can be performed by using a commercially available "lure adaptor". The lure adaptor is normally designed so that a needle tube at one end of a needle of about 21G in thickness is covered by an elastic cap (hemostatic rubber cover) such as a rubber or the like and the other end is constructed by a lure tapered type needle hub. A normal injection needle is joined to the lure tapered type needle hub, thereby making it possible to perform vacuum blood collection using an injection needle. Accordingly, sting based on a narrow needle can be performed at a side where the needle is stung into a human body, and also sting based on a thick needle of 21G can be performed at a side (lure adaptor side) where the needle is stung through the plug body of the blood collecting tube.

However, according to the method using the lure adaptor, a space of about 100µL exists between the joint portions of the injection needle and the lure adaptor, and it serves as a dead volume of blood when blood is collected. Therefore, it is necessary to excessively collect blood by the amount corresponding to the dead volume, and thus an useless amount of blood occurs when blood is collected. This becomes more critical particularly when a small amount of blood is collected. Furthermore, the pressure reduction degree of the vacuum blood collecting tube is lowered by the amount corresponding to the dead volume, and thus there is a problem that the blood collecting speed is lowered, etc. when a small amount of blood is collected. Furthermore, there are also problems in workability and cost, for example, a work of joining the lure adaptor and the injection needle is required before blood collection, and the cost of the lure adaptor is increased.

An object of the present invention is to provide a blood collecting needle that provides little pain to a person being examined, can be stung through the plug body of a blood collecting tube, and is excellent in workability and cost.

According to the invention, this object is achieved by a blood collecting needle as defined by claims 1. The dependent claims define preferred embodiments of the invention.

The above object of the present invention can be attained by the following construction.
(1) A blood collecting needle, which comprises:
   a needle hub;
   a human-body puncturing needle tube provided at one side of the needle hub; and
   a plug-body puncturing needle tube provided at the other side of the needle hub,
   wherein an outer diameter of a tip of the human-body puncturing needle tube and an outer diameter of a tip of the plug-body puncturing needle tube are different from each other.
(2) The blood collecting needle as described in (1) above,
   wherein the outer diameter of the tip of the plug-body puncturing needle tube is larger than the outer diameter of the tip of the human-body puncturing needle tube.
(3) The blood collecting needle as described in (1) or (2) above,
   wherein the needle hub is provided with a mark indicating an orientation of a blade.
(4) The blood collecting needle as described in any of (1) to (3) above,
   wherein the outer diameter of the tip of the human-body puncturing needle tube is 0.42mm or less.
(5) The blood collecting needle as described in any of (1) to (4) above,
   wherein the outer diameter of the tip of the human-body puncturing needle tube is in a range of from not less than 0.26mm to not more than 0.34mm, and an inner diameter of the tip of the human-body puncturing needle tube is 0.17mm or more.
(6) A test kit, which comprises:
   a blood collecting needle as described in any of (1) to (5) above; and
   a test chip.
(7) A blood collecting kit, which comprises:
   a blood collecting needle as described in any of (1) to (5) above; and
   a vacuum blood collecting tube.
(8) The blood kit as described in (7) above,
   wherein the vacuum blood collecting tube has an interior content of 1.5mL or less.

### Brief Description of the Drawings

Fig. 1 is a schematic diagram showing the shapes of general blood collecting needles;

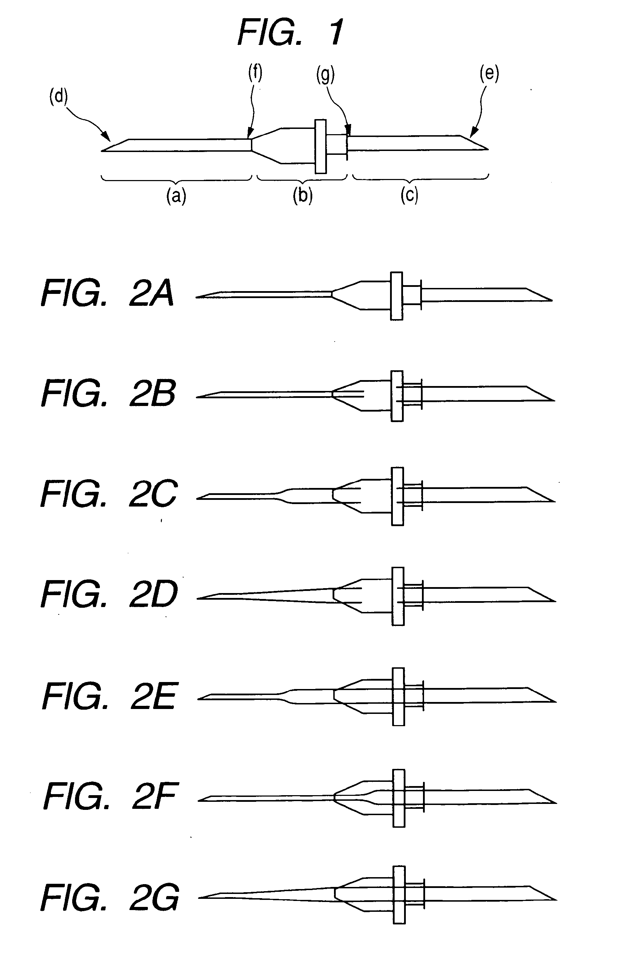

Fig. 2A is an outlook diagram of a blood collecting needle of the present invention, Fig. 2B is a diagram showing an embodiment of the blood collecting needle of the present invention, Fig.2C is a schematic diagram showing an embodiment of the blood collecting needle of the present invention, Fig.2D is a diagram showing an embodiment of the blood collecting needle of the present invention, Fig.2E is a schematic diagram showing an embodiment of the blood collecting needle of the present invention, Fig.2F is a schematic diagram showing an embodiment of the blood collecting needle of the present invention, and Fig.2G is a schematic diagram showing an embodiment of the blood collecting needle of the present invention; and
Fig. 3 is a diagram showing a cut shape of the needlepoint in the puncturing needle of the present invention,
wherein (a) denotes human-body puncturing needle tube; (b) denotes needle hub (hub); (c) denotes plug-body puncturing needle tube; (d) denotes tip of human-body puncturing needle tube; (e) denotes tip of plug-body puncturing needle tube; (f) denotes base end of human-body puncturing needle tube; (g) denotes base end of plug-body puncturing needle tube; a denotes first polishing angle; and b denotes second polishing angle.

### Detailed Description of the Invention

The present invention will be described hereinafter in detail.

### <Purpose of use of blood collecting needle>

The blood collecting needle of the present invention is suitably used when blood of humans and other animals is collected. The collected blood is normally used for blood examination.

### <Classification of puncture needle>

In order to collect blood and analyze the components of the blood, a hollow type needle and a solid type needle are known as a needle for collecting blood from an arm, an elbow, a fingertip or the like.

For example, a hollow type puncture needle is generally used in a medical institution or for health diagnosis so that it is stung into vein to collect blood in the vein and the components of the blood are generally analyzed. For example, it is used to analyze components for diagnosing diabetes such as blood sugar, hemoglobin A1c (HbA1c) or the like, components for diagnosing hepatic function such as glutamate pyruvate transaminase (GPT), glutamate oxaloacetate transaminase (GOT) or the like, and components for diagnosing function of kidney such as creatinine (CRE), urea nitrogen (BUN) or the like.

On the other hand, a solid puncture needle which is called as lancet is used so that a person being examined punctures his/her fingertip to collect blood in a device for self-measuring blood sugar.

The present invention relates to a blood collecting needle which has a hollow portion in the needle.

### <Blood collecting needle>

The blood collecting needle is generally constructed to have a needle tube (cannula) and a needle hub (hub), and the needle tube (cannula) penetrates through the needle hub (hub), and has blade edges at both the ends thereof. The general structure of the blood collecting needle is shown in Fig. 1. In Fig. 1, the needle tube penetrates through the inside of "needle hub (b)". At the human-body puncture side of the needle tube, the site extending from "the base end (f) of a human-body puncturing needle tube" as a start point till "the tip (d) of the human-body puncturing needle tube" is set as "a human-body puncturing needle tube (a)", and it has a blade edge at the tip (d). Furthermore, at the plug-body puncture side, the site extending from the "base end (g) of a plug-body puncturing needle tube" as a start point till "the tip (e) of the plug-body puncturing needle tube" is set as "a plug-body puncturing needle tube (c)", and it has a blade edge at the tip (e).

As a continuous blood collecting needle is generally known a blood collecting needle having a hemostatic rubber cover at the plug-body puncture side of the needle tube. A general style is defined in JIS T-3220. Normally, the blood collecting needle is normally sold in the market while covered by a protection cap (protector) (not shown in Fig. 1) for protecting the blade. When blood is collected, the blood collecting needle is mounted to a vacuum blood collecting holder so that the recess side of the vacuum blood collecting holder faces the plug-body puncturing needle tube (c) side. Thereafter, the human-body puncturing needle tube (a) is stung from the tip (d) thereof into vein and further a vacuum blood collection tube is put into the holder, whereby the plug-body puncturing needle tube (c) is stung from the tip (e) thereof into the plug body of the vacuum blood collecting tube and blood collection is started. Normally, the blood collection needle is manufactured according to the following method. That is, both the ends of one needle tube is polished to create blades, and then a needle hub (b) is joined to the center portion of the needle tube to which adhesive agent adheres, whereby the needle tube and the needle hub are adhesively attached to each other. Thereafter, the hemostatic cover is covered on the plug-body puncturing needle tube (c).

According to the blood collection needle of the present invention, the tip (d) of the human-body puncturing needle tube and the tip (e) of the plug-body puncturing needle tube are different from each other in outer diameter. It is preferable that the outer diameter of the tip (e) is larger than the outer diameter of the tip (d).

Furthermore, in order to prevent a needle stick accident or infection of a blood-collected person or a transactor for medical waste, a cover formed of rubber or the like may be provided on the plug-body puncturing needle, or a holder as a guide when a vacuum blood collecting tube is used may be provided.

In the needle tube of the blood collecting needle of the present invention, two needle tubes different in outer diameter may be used for "the human-body puncturing needle tube (a) " and "the plug-body puncturing needle tube (c)" respectively, or one needle tube which is varied in outer diameter at some midpoint thereof may be used for both "the human-body puncturing needle tube (a)" and "the plug-body puncturing needle tube (c)". Fig. 2A is a diagram showing the outlook of a blood collecting needle, and the tip (d) of the human-body puncturing needle tube (a) and the tip (e) of the plug-body puncturing needle tube (c) are designed as sharp blades. Figs. 2B, 2C and 2D show examples of the blood collection needle of the present invention when two needle tubes different in outer diameter are used. Figs. 2E, 2F and 2G show examples of the blood collection needle of the present invention when one needle tube varied in outer diameter at some midpoint is used.

When two needle tubes different in outer diameter are used, the base end (f) of the human-body puncturing needle tube (a) and the base end (g) of the plug-body puncturing needle tube (c) are air-tightly and water-tightly mounted on the needle hub (b) so that they do not drop out of the needle hub. The needle hub of a normally used blood collecting needle is designed so as to be equipped with each of needle tube insertion ports having the same diameter at each of right and left sides thereof. However, according to the needle hub of the blood collecting needle of the present invention, it is preferable that the needle tube insertion ports at the right and left sides of the needle hub are different from each other in outer diameter so that needle tubes different in diameter are inserted into the needle tube insertion ports. In connection with this structure, the flow paths at the portions of the needle hub into which the needle tubes are inserted are different in diameter between the right and left sides. A portion for joining the right and left flow paths into which the needle tubes are inserted serves a blood flow-in portion at which blood comes into direct contact with the needle hub. It is preferable that the inner wall of the blood flow-in portion of the needle hub (b) is smooth so that blood quickly moves from the human-body puncturing needle tube (a) to the plug-body puncturing needle tube (c) when blood is collected. The shape of the human-body puncturing needle tube (a) may be set so that the outer diameter is substantially equal in the area from the tip (d) of the needle till the base end (f) of the needle as shown in Fig. 2B, or so that the outer diameter is varied like a tapered shape in the area from the tip (d) of the needle till the base end (f) of the needle as shown in Fig. 2D. Furthermore, the needle may be designed in such a step structure having a straight shape that the outer diameter is different between the tip (d) side and the base end (f) side as shown in Fig. 2C.

When one needle tube varied in outer diameter thereof at some midpoint thereof is used, the needle may be designed so that the outer diameter thereof varies like a tapered shape from the tip (d) of the human-body puncturing needle tube (a) till the tip (e) of the plug-body puncturing needle tube (c) as shown in Fig. 2G, or may be designed in such a step structure having a straight shape that the tip (d) side and the tip (e) side are different in outer diameter from each other as shown in Figs. 2E and 2F. At this time, the needle hub (b) may be provided to the straight structure portion as shown in Fig. 2E, or the needle hub may be provided to the step portion through which the straight structures are joined as shown in Fig. 2F.

When it is considered that the blood collecting needle is produced at a low price, it is preferable to produce a blood collecting needle in which two needle tubes different in outer diameter are used and the outer diameter is substantially equal from the tip (d) of the needle tube of the human-body puncturing needle tube (a) till the base end (f) as shown in Fig. 2B.

From the viewpoint of pain, the outer diameter of the tip (d) of the human-body puncturing needle tube (a) is set preferably to 0.42mm or less, more preferably to 0.38mm or less, and most preferably to 0.34mm or less. Furthermore, from the viewpoint of the strength of the needle, it is preferably set to 0.26mm or more.

Furthermore, from the viewpoint of hemolysis and blood collecting speed, the inner diameter is preferably set to 0. 17mm or more. The hemolysis will be described later.

The length of the human-body puncturing needle tube (a) is preferably set to 5mm to 38mm, more preferably to 7mm to 25mm, and most preferably to 9mm to 20mm. The length of the human-body puncturing needle tube (a) corresponds to the length from the base end (f) to the tip (d) except for the portion hidden by the needle hub (b).

The outer diameter of the tip (e) of the plug-body puncturing needle tube (c) is preferably set to 0.40mm or more because of easiness of puncturing into the plug body (rubber plug or the like), more preferably to 0.50mm or more, and most preferably to 0.60mm or more.

It is better that the length of the plug-body puncturing needle tube (c) is longer from the viewpoint that the needle tube has a length enough to penetrate through the plug body of the vacuum blood collecting tube. However, it is better that the length of the needle tube (c) is shorter from the viewpoint that the tip (e) of the plug-body puncturing needle tube (c) is not brought into contact with blood stocked in the blood collecting tube. The length of the plug-body puncturing needle tube (c) is preferably set to 10mm to 25mm, more preferably to 12mm to 22mm and most preferably to 14mm to 20mmm. The length of the plug-body puncturing needle tube (c) indicates the length from the base end (g) to the tip (e) except for the portion hidden by the needle hub (b).

The difference between the outer and inner diameters of the needle tube is preferably set to a smaller value because the inner diameter can be set to a larger value and thus the damage imposed on the whole blood is small. On the other hand, the difference concerned is preferably set to a larger value in order to keep the dynamical strength of the needle. In this invention, the difference between the outer and inner diameters of the needle tube is preferably set in the range from not less than 0.05mm to not more than 0.17mm, and more preferably set in the range from not less than 0.08mm to not more than 0.14mm. The difference between the outer and inner diameters may be different or equal between the human-body puncturing needle tube (a) and the plug-body puncturing needle tube (c).

The outer diameter of the human-body puncturing needle tube (a) of the blood collecting needle of the present invention is preferably smaller as compared with the needle of 21G which is used for normal blood collection. Therefore, the orientation of the blade of the needle is hard to see, and thus a mark indicating the orientation of the blade is preferably provided to the needle hub (b). This mark may be a mark such as a triangle, a circle, a rectangle or the like, or it may be a projection or recess so that it can be identified by merely touching it. Furthermore, the mark may be provided by printing, a seal putting method or the like.

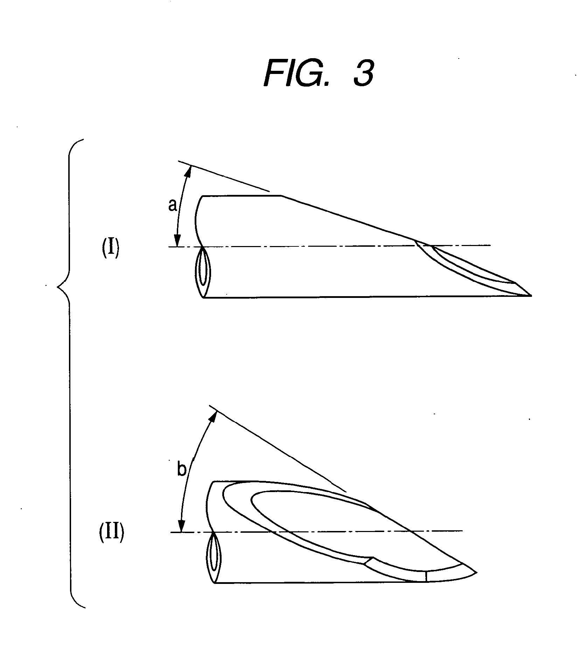

The cutting shape of the tip of the needle for puncture may be set to a three-side cut or one-side cut shape or a linear cut or curved-line cut shape. With respect to the cut angle of the needlepoint in the case of the one-side cut shape, it is preferable that the angle θ of the blade edge defined in Japanese Industrial Standards T3101-1979 ranges from 8 degrees to 30 degrees. With respect to the cut of the needlepoint in the case of the three-side cut shape, it is preferable that the first polishing angle a is set in the range from 7 degrees to 20 degrees and the second polishing angle b is set in the range from 10 degrees to 30 degrees. Fig. 3 shows the needlepoint of a preferable three-side cut shape. The first polishing angle a is shown in (I) of Fig. 3, and the second polishing angle b is shown in (II) of Fig. 3 (see ISO7864).

### (raw material of needle)

The material of the blood collecting needle of the present invention may be stainless steel such as SUS304, metal such as nickel free stainless or the like, resin such as polycarbonate, acrylate type polymer, inorganic material such as quartz glass, boron silicate glass or the like, and it is not limited to specific material insofar as it produces a hollow needle. It is preferable to use metal such as stainless or the like because it is easily to keep the dynamic strength at the outer diameter described above. Furthermore, the outer surface or inner surface of the blood collecting needle may be subjected to coating or other treatments by using material different form the material of the needle. Particularly, another material (silicon or the like) may be coated on the needle to reduce the puncture resistance and thus make the puncture easy.

Furthermore, the blood collecting needle of the present invention may be subjected to various kinds of improvements considering safety, workability, etc. For example, it is preferable to provide a safe cover for locking the needle with a cover so that any one is prevented from touching the needle after blood collection. Furthermore, it is preferable that the needle hub is made transparent or semi-transparent so that sting into vein can be identified, that is, so-called flashback can be checked with eyes.

Furthermore, the blood collecting needle may be mounted on a holder for vacuum blood collection to assemble a integral type holder having a needle. By providing an integral type blood collecting instrument as a use style in advance, there can be omitted a labor of mounting a needle when blood collection is carried out and detaching the needle after the blood collection is carried out, and further a needle puncturing accident hardly occurs, so that the blood collecting time can be particularly shortened in a hospital or the like which contains a large number of persons who are subjected to blood collection.

### (Production of Needle)

The blood collecting needle of the present invention is not limited to a special one, and it can be produced according to the normal method of this field.

### [hemolysis]

In general, the collected blood is subjected to centrifugal separation at a rotational number of about 3000 rpm for 10 minutes to precipitate hematocyte components, and then supernatant fluid is collected to achieve blood plasma and blood serum. The analysis and diagnosis are carried out by using the blood plasma and the blood serum thus achieved. However, when strong force such as pressure or the like is applied to the collected whole blood, blood erythrocyte may be broken and thus blood pigment in the blood erythrocyte is eluted, so that the blood plasma and the blood serum are tinged with red. This is called as hemolysis.

When test substance such as hemolyzed blood plasma, blood serum or the like is used for analysis and diagnosis, it may affect the analysis result. In the case of components that exist in higher concentration in blood erythrocyte than in blood plasma, the above phenomenon appears more remarkably, so that deviation may be induced in the analysis result. Such a component is potassium ions, lactate dehydrogenase (LDH), adenylate kinase (AK), catalase or the like.

The blood collecting needle is used for blood collection based on the vacuum blood collecting method used in the vacuum blood collecting tube.

### <Vacuum Blood Collecting Tube>

The blood collecting needle of the present invention may constitute a blood collecting kit in combination with a vacuum blood collecting tube. A holder for a vacuum blood collecting tube is mounted on a blood collecting tube, and the vacuum blood collecting tube is connected to the holder, whereby the collection of the blood is started by negative pressure of the blood collecting tube.

The vacuum blood collecting tube used when the blood collection is carried out by using the blood collecting tube of the present invention may be any commercially-available vacuum blood collecting tube.

Anticoagulant, glycolytic inhibitor or the like is normally sealingly filled in the vacuum blood collecting tube in advance, and it is selectively used in accordance with the component to be analyzed. Furthermore, with respect to the amount of blood to be collected by a blood collecting tube, vacuum blood collecting tubes different in capacity are selectively used in accordance with the analysis object. Blood collecting tubes of 2mL, 5mL and 10mL in blood collection amount are generally used. The time required for blood collection is dependent on the status of a person being examined (generally, patient), the skill degree of an examiner (generally, a doctor, a nurse, a clinical laboratory technologist), and the blood collection is normally completed in the range from several seconds to several tens seconds.

The pressure reduction degree of the commercially-available vacuum blood collecting tube ranges from about -200mmHg to about -650mmHg, and any commercially available vacuum blood collecting tube may be used in the blood collecting kit of the present invention. In consideration of the blood collecting speed, etc., the vacuum blood collecting tube has preferably a pressure reduction degree of -400mmHg or more, more preferably a pressure reduction degree of -450mmHg or more, and most preferably a pressure reduction degree of -500mmHg or more. The pressure reduction degree described here is set on the assumption that the atmospheric pressure is defined as 0mmHg and the vacuum is defined as -760mmHg.

In this invention, when a blood collecting tube having a small capacity is used in conformity with collection of a small amount of blood, it is possible to collect blood by only the amount required for examination, and thus the load imposed on the person being examined can be reduced. It is preferable to use a blood collecting tube of 7mL or less in capacity, more preferable to use a blood collecting tube of 4mL or less in capacity and most preferably to use a blood collecting tube of 1.5mL or less.

### <Test Chip>

The puncturing needle of the present invention can constitute a test kit in combination with a test chip. As compared with blood examination using an automatic analysis device which has been hitherto carried out in a medical institution/examination institution, the examination based on the examination chip enables many items of examination with a small amount of blood. Assuming that the time required for blood collection is fixed, as the amount of blood required for examination is smaller, the inner diameter of the blood collecting needle of the present invention can be reduced and thus the outer diameter of the needle can be reduced. Therefore, the blood collecting needle of the present invention is preferably used in combination with the examination chip which can perform examination with a small amount of blood.

The examination chip means a compact chip type device for performing blood analysis, and according to this device, in order to measure various kinds of components contained in blood, analyte such as blood is made to flow through a flow path having a minute cross-section area by using capillary phenomenon or electrophoresis, and reacted with reagent. Thereafter, the respective components in the blood are separated from one another, and then transmission spectroscopic analysis is carried out, or light emission reaction with reagent is induced and then emitted light is spectroscopically analyzed.

Furthermore, the puncturing needle of the present invention may be used in other applications than the application of puncturing the vein of a human to achieve blood. Particularly, it is effective to a case where blood is collected from a small animal having a narrow blood vessel, and it is used when blood is collected from animals such as a dog, a cat, etc. in an animal hospital. Furthermore, it may be used when blood is collected from small animals such as a rat, a mouse, a rabbit, etc. in physical and chemical experiments of chemistry, biochemistry, biology, etc.

Furthermore, even in the blood collection targeting humans, the needle of the present invention can be used not only in hospitals in which normal blood collection is carried out and for health diagnosis and complete physical examinations, but also for blood collection to monitor health in drugstores, health care shops, etc. As compared with the normal blood collecting needle, the pain can be reduced and thus the needle of the present invention is more effective to applications such as health check, etc. for healthy persons, particularly persons who look healthy, but are nervous about diseases. As measurements targeting healthy persons may be considered not only general biochemical items/blood count, but also a measurement of fluidity (smoothness) of blood, a measurement of stress marker, etc. The fluidity (smoothness) of blood can be measured by MC-FAN (produced by MC Research Company) or the like. The puncturing needle of the present invention brings little pain at the blood collection time, and thus it is effective to measurements of materials which may be affected by an impulse under blood collection, such as a stress marker or the like.

### [Embodiments]

The present invention will be described hereunder by using embodiments, however, the present invention is not limited to these embodiments.

### [Production of two-needle joint type blood collecting needle a]

According to a method of cold-stretching a commercially available hollow stainless tube and processing one needlepoint into a incisive shape by three-side cutting while the angle of the first polishing angle of one needlepoint is set to 9 to 13 degrees, there are prepared two needle tubes of a plug-body puncturing needle tube of 0.81mm in outer diameter and a human-body puncturing needle tube of 0.31mm in outer diameter. The two needle tubes are joined to a needle hub of resin for a blood collecting needle by adhesive agent, and then a rubber cover is mounted on the plug-body puncture side. The result is subjected to a sterilization treatment by gamma rays and two joint blood collecting needles a of an embodiment I are prepared. The blood collecting needle a has the structure shown in Fig. 2B.

### [Production of blood collecting needle having the step structure]

According to a dice-based drawing process, a pipe having a step structure in which the outer diameter of one end thereof is equal to 0.45mm and the outer diameter of the other end thereof is equal to 0.31mm is prepared from a commercially available hollow stainless tube, and then processed into a incisive shape by three-side cutting while the angle of the first polishing angle of both the ends of the needle tube is set to 9 to 13 degrees. The needle tube having the step structure is joined to the member of the needle hub of resin for the blood collecting needle by adhesive agent, and then a rubber cover is mounted on the larger outer diameter (0.45mm) side. Then, the result is subjected to a sterilization treatment by gamma rays, and a blood collecting needle b having the step structure of an embodiment 2 is prepared. The blood collecting needle b has the structure shown in Fig. 2F.

### [Production of straight blood collecting needle c]

A commercially available hollow stainless tube is cold-stretched, and processed into an incisive shape by three-side cutting while the angle of the first polishing angle of the needlepoint of each of both the ends is set to 9 to 13 degrees, thereby preparing a needle tube of 0.31mm in outer diameter. The needle tube is joined to the member of the needle hub of resin for the blood collecting needle by adhesive agent, and a rubber cover is mounted on the plug-body puncture side. Then, the result is subjected to a sterilization treatment by gamma rays, and a straight blood collecting needle c of a comparative example 1 is prepared. The blood collecting needle c has the structure of a general straight blood collecting needle.

### [Production of injection needle e]

A commercially available hollow stainless tube is cold-stretched, and processed into an incisive shape by three-side cutting while the angle of the first polishing angle of one needlepoint is set to 9 to 13 degrees, thereby preparing a needle tube for an injection needle of 0. 31mm in outer diameter. The needle tube is joined to the member of the needle hub of resin for the injection needle by adhesive agent, and subjected to a sterilization treatment by gamma rays, thereby preparing an injection needle e of a comparative example 3. The injection needle e has the structure of a general straight injection needle.

### [Commercially available blood collecting needle]

A commercially available blood collecting needle (21G) based on ISO9626:1991 is prepared as the blood collecting needle d of a comparative example 2. The needle tube is cut, a photograph of the cross-section of the cut needle tube is taken by using an optical microscope (50 times), and the dimensions of the outer diameter and the inner diameter are measured on the basis of the image.

The dimensions of the respective needle tubes are shown in table 1. In table 1, the length corresponds to the length of the portion exposed to the outside except for the portion hidden by the needle hub.

**Table 1**

| | | FOR HUMAN-BODY PUNCTURE | | | FOR PLUG-BODY PUNCTURE | | |
|---|---|---|---|---|---|---|---|
| | | OUTER DIAMETER OF TIP (mm) | INNER DIAMETER OF TIP (mm) | LENGTH (mm) | OUTER DIAMETER OF TIP (mm) | INNER DIAMETER OF TIP (mm) | LENGTH (mm) |
| EMBODIMENT 1 | TWO-NEEDLE JOINT TYPE BLOOD COLLECTING NEEDLE a | 0.31 | 0.22 | 15 | 0.81 | 0.56 | 15 |
| EMBODIMENT 2 | STEP STRUCTURE BLOOD COLLECTING NEEDLE b | 0.31 | 0.20 | 15 | 0.45 | 0.34 | 15 |
| COMPARATIVE EXAMPLE 1 | STRAIGHT BLOOD COLLECTING NEEDLE c | 0.31 | 0.22 | 15 | 0.31 | 0.22 | 15 |
| COMPARATIVE EXAMPLE 2 | COMMERCIALLY AVAILABLE BLOOD COLLECTING NEEDLE (21G) d | 0.81 | 0.56 | 36 | 0.81 | 0.56 | 17 |
| COMPARATIVE EXAMPLE 3 | INJECTION NEEDLE e | 0.31 | 0.22 | 15 | - | - | - |

Each of the blood collecting needles a to d is mounted in a holder for the vacuum blood collecting tube [Venoject II holder S/produced by Terumo Corporation] and used for experiments. The injection needle e is mounted in a commercially available [Venoject II lure adaptor S¥produced by Terumo Corporation], joined to a holder for vacuum blood collecting tube [Venoject II holder S/produced by Terumo Corporation], and used for experiments. The outer diameter of the needle tube of the lure adaptor is equal to 0.81mm.

### [Estimation of plug-body puncturing performance]

The vacuumblood collecting tube is inserted into the holder having the blood collecting needle mounted therein at 20 times. The estimation is made by indicating "B" in the case where the needle is bent when the needle is stung into the rubber plug, and indicating "A" in the case where the needle can be stung into the rubber plug with no problem at all times. The result is shown in Table 2. ten [Venoject II vacuum blood collecting tube, heparin Li 5mL/produced by Terumo Corporation] and ten [neotube PET, heparin Li 4mL/produced by Nipro Corporation] are used as the vacuum blood collecting tubes.

In the blood collecting needles of the embodiments 1 and 2 and the comparative example 2, the outer diameter of the needle tube at the plug-body puncturing side is large, and they can be stung into the rubber plug with no problem. Furthermore, in the comparative example 3 in which the lure adaptor is mounted on the injection needle, the outer diameter of the lure adaptor is large, and thus it can be stung with no problem. On the other hand, in the straight blood collecting needle c of the comparative example 1, the outer diameter of the needle tube at the plug-body puncturing side is equal to 0.31mm and thus small, so that it is bent or hardly stung in some cases when it is stung into the rubber plug.

### [Estimation of Pain]

Ten able-bodied persons are punctuated with a holder having a blood collecting needle mounted therein to estimate whether the persons feel pain. one score would be set if a person feels pain while zero score would be set if a person feels no pain, and the estimations of the ten persons are summed. Nine to ten scores are estimated as C, seven to eight scores are estimated as B, and six scores or less are estimated as A. The estimation result is shown in Table 2.

In the embodiments 1 and 2 and the comparative examples 1 and 3, the outer diameter of the needle tube at the human-body puncturing side is small, and the persons feel little pain. On the other hand, in the commercially available blood collecting needle d of the comparative example 2, the outer diameter of the human-body puncturing side is also large, and thus the persons feel pain at all times.

### [Workability]

The vexatiousness of the work from the take-out of the blood collecting needle till the puncture is estimated as workability. In the blood collecting needles of the embodiments 1 and 2 and the comparative examples 1 and 2, the blood collecting needle is merely stung after it is mounted on the holder (the result is indicated by A in table 2). However, in the case of the injection needle of the comparative example 3, the injection needle is mounted on the lure adaptor, further mounted on the holder and then stung. Therefore, the operation till the blood collection is cumbersome. Furthermore, the cost of the lure adaptor is increased, and the lure adaptor must be wasted, so that this blood collecting needle is disadvantageous in cost, environment, etc. as compared with the blood collection using the blood collecting needle (the result is indicated by B in table 2).

**Table 2**

| | | PLUG-BODY PUNCTURING PERFORMANCE | PAIN | WORKABILITY |
|---|---|---|---|---|
| EMBODIMENT 1 | TWO-NEEDLE JOINT BLOOD COLLECTING NEEDLE a | A | A | A |
| EMBODIMENT 2 | STEP STRUCTURE BLOOD COLLECTING NEEDLE b | A | A | A |
| COMPARATIVE EXAMPLE 1 | STRAIGHT BLOOD COLLECTING NEEDLE c | B | A | A |
| COMPARATIVE EXAMPLE 2 | COMMERCIALLY AVAILABLE BLOOD COLLECTING NEEDLE d (21G) | A | C | A |
| COMPARATIVE EXAMPLE 3 | INJECTION NEEDLE e + LURE ADAPTOR | A | A | B |

In the blood collecting needle of the embodiments 1 and 2 in which the outer diameter of the tip of the human-body puncturing needle tube is small and the outer diameter of the tip of the plug-body puncturing needle tube is large, it can be stung into the rubber plug with no problem, and pain is little when blood is collected. On the other hand, in the straight blood collecting needle of the comparative example 1, pain is little when blood is collected, however, there is a case where the needle is bent when the needle is stung into the rubber plug. Furthermore, in the commercially available blood collecting needle of the comparative example 2, the outer diameter is large, and pain is strong. In the method of mounting the lure adaptor on the injection needle in the comparative example 3, the human-body puncture side can be narrowed while the plug-body puncturing side is thickened. However, the preparation till blood collection is cumbersome, and it is disadvantageous in cost and environment.

### [Estimation of hemolysis]

Three able-bodied persons are subjected to blood collection by using the blood collecting needle/injection needle of the embodiments/comparative examples and [the Venoject II vacuum blood collecting tube/heparin Li 5mL/produced by Terumo Corporation] to estimate the hemolysis. The whole blood achieved is subjected to centrifugal separation at 3000rpm at room temperature for 10 minutes and supernatant fluid is withdrawn. The, absorption spectrum of the supernatant fluid is measured by UV-2550 (produced by Shimadzu Corporation) and the hemolysis is estimated on the basis of the increase of the absorbance of 415nm originated from the absorption of hemoglobin. As a comparative example, the whole blood which is pooled in a Sumiron tube and not passed through the hollow needle is subjected to centrifugal separation under the same condition to achieve blood plasma, the absorbance of the blood plasma thus achieved is measured, and the increment of OD with respect to the absorbance of the blood plasma is estimated. The increment of OD is defined as the degree of hemolysis, and if there is an increment of OD=0.1 or more, "hemolysis" is judged. In all the blood collecting needles of the embodiments 1 and 2 and the comparative examples 1 to 3, it is judged that no hemolysis occurs in the blood after the blood collection.

According to the present invention, there can be provided a blood collecting needle that brings a person being examined with little pain, can be smoothly stung into a plug body of a blood collecting tube and is excellent in workability and cost.

The entire disclosure of each and every foreign patent application from which the benefit of foreign priority has been claimed in the present application is incorporated herein by reference, as if fully set forth.

## Claims

1. A blood collecting needle, which comprises:
a needle hub (b);
a human-body puncturing needle tube (a) provided at one side of the needle hub (b); and
a plug-body puncturing needle tube (c) provided at the other side of the needle hub (b),
wherein an outer diameter of a tip (d) of the human-body puncturing needle tube (a) and an outer diameter of a tip (e) of the plug-body puncturing needle tube (c) are different from each other.

2. The blood collecting needle according to claim 1,
wherein the outer diameter of the tip (e) of the plug-body puncturing needle tube (c) is larger than the outer diameter of the tip (d) of the human-body puncturing needle tube (a).

3. The blood collecting needle according to claim 1 or claim 2,
wherein the needle hub (b) is provided with a mark indicating an orientation of a blade.

4. The blood collecting needle according to any one of claims 1-3,
wherein the outer diameter of the tip (d) of the human-body puncturing needle tube (a) is 0.42mm or less.

5. The blood collecting needle according to any one of claims 1-4,
wherein the outer diameter of the tip (d) of the human-body puncturing needle tube (a) is in a range of from not less than 0.26mm to not more than 0.34mm, and an inner diameter of the tip (d) of the human-body puncturing needle tube (a) is 0.17mm or more.

6. A test kit, which comprises:
a blood collecting needle according to any one of claims 1-5; and
a test chip.

7. A blood collecting kit, which comprises:
a blood collecting needle according to any one of claims 1-5; and
a vacuum blood collecting tube.

8. The blood kit according to claim 7,
wherein the vacuum blood collecting tube has an interior content of 1.5mL or less.
